# EUROPEAN PATENT SPECIFICATION

(11) **EP 3 236 886 B1**
(45) Date of publication and mention of the grant of the patent: **12.08.2020**
(21) Application number: 15817535.6
(22) Date of filing: 23.12.2015
(51) Int. Cl.: A61F 2/34, A61F 2/46, A61F 2/30

(54) **PROSTHESIS**
PROTHESE
PROTHÈSE

(30) Priority: 24.12.2014 GB 201423179
(43) Date of publication of application: 01.11.2017
(73) Proprietor: MatOrtho Limited, Leatherhead Surrey KT22 7BA (GB)
(72) Inventor: BIRD, Timothy Alan, Westbury Wiltshire BA13 3NS (GB); TAYLOR, Andrew Clive, Chichester West Sussex PO18 0AW (GB); COLLINS, Simon, Tetbury Gloucestershire GL8 8NT (GB); TUKE, Michael Anthony, Leatherhead Surrey KT22 7BA (GB)
(74) Representative: Hocking, Adrian Niall
(86) International application number: PCT/GB2015/054161
(87) International publication number: WO 2016/102976

(56) References cited:
- DE-U1-202008 015 356
- FR-A1- 2 911 062
- US-A1- 2012 053 699
- US-A1- 2013 282 133

## Description

The present invention relates to a prosthesis. More particularly, it relates to an acetabular hip prosthesis.

The efficient functioning of the hip joint is important to the well-being and mobility of the human body. Each hip joint is comprised of the upper portion of the femur which terminates in an offset bony neck surmounted by a ball-shaped portion, known as the femoral head, which rotates within the acetabulum of the pelvis. Diseases such as rheumatoid- and osteoarthritis can cause deterioration and erosion of the cartilage lining of the acetabulum so that the femoral head and the hip bone rub together causing pain and further erosion. Bone erosion may cause the bones themselves to attempt to compensate for the erosion which may result in the bone becoming misshapen.

Operations to replace the hip joint with an artificial implant are well-known and widely practiced. Generally, the hip prosthesis will be formed of two components, namely: an acetabular component which lines the acetabulum; and a femoral component which replaces the femoral head. The femoral component may be total femoral head replacement in which the component includes a head, and a stem and a neck connecting the head to the stem. In use the stem is inserted into the medullary canal of a prepared femur. Alternatively, where appropriate, the femoral head component may be a resurfacing prosthesis which is attached to a suitably machined femoral head. This resurfacing arrangement does not invade the medullary canal and is generally more bone conserving.

In an operation to insert a prosthetic acetabulum into a patient's pelvis the surgeon first uses a reamer to cut a cavity in the patient's pelvis. An acetabular cup is then inserted into the cavity. Normally, it is desirable to retain as much of the original healthy bone surface as possible.

The acetabulum is surrounded by a ring of cartilage known as the acetabular labrum. In general, the labrum is about 2 to 3 mm thick. However, its size is not constant throughout and it is generally wider and thinner in the anterior region.

The labrum serves to increase the overall depth of the acetabulum and thereby make it more difficult for the head of the femur to move out of the desired position in the acetabulum. In addition, the labrum acts to protect the pelvis from damage from the femoral head.

Unfortunately, the preparation of the acetabulum for insertion of the acetabular cup prosthesis generally compromises or even fully removes the labrum.

This means that after the insertion of the prosthesis, the functions of the labrum are not performed and as such the general post-operative structure of the joint does not mirror the natural configuration.

Once the acetabulum has been prepared, the acetabular cup prosthesis can be inserted. In one arrangement, the acetabular cup prosthesis may be fabricated from polyethylene. These cups are general cemented into the acetabulum and require only light pressure to seat them in the bone cement which is used to fix the prosthesis in position. Generally, polymethylmethacrylate bone cement is used.

One alternative type of acetabular cup prosthesis has a polyethylene liner for articulation with the femur and a metal shell in which the liner sits which is inserted into the pelvic cavity. The shell is first located in the acetabulum and the liner is then placed in the shell. The metal shell may be implanted without cement such that it relies on a jamb fit between the metal shell and the patient's acetabulum to provide primary fixation. However, in some arrangements, screws may be used to secure the cup shell in position in the pelvis before the liner is placed in position. The insertion of the metal shell into the pelvis requires considerable force. As the surgeon applies this force, there is a risk that the metal shell can become damaged or deformed. There is also a possibility that during the application of the force, the shell may move so that it is not in the optimum alignment in the acetabulum. Often the metal shells have outer surfaces or coatings which encourage bone to grow into them over time; this biological fixation is often referred to as secondary fixation.

With this type of prosthesis, the polyethylene liner unit is generally snapped or screwed into the metal shell after the metal shell has been seated in the acetabulum. Thus the inner surface of the liner forms the socket part of the joint.

More recently, ceramics have been used as an alternative to the plastics liner as there have been suggestions that the use of the plastic liner may create wear debris during use which can lead to some bone death. In this arrangement, the metal shell is inserted into the pelvis and the ceramic liner is then inserted into the shell. However, as discussed in EP2174621 there are problems associated with the insertion of this ceramic liner and in achieving an accurate fit between the liner and the cup. There have therefore been suggestions of using preassembled acetabular cups with ceramic liners whereby it is the combination of the cup and liner which is inserted into the acetabulum.

There has also been a suggestion that the cups should be made substantially completely from ceramic, that is to say that the cups do not require a metal shell or they have a metal shell which is sufficiently thin that it cannot support being introduced separately into the pelvis. However, the use of a ceramic acetabular hip prosthesis may suffer from various disadvantages. In particular, the ceramic, which is either unsupported by a shell or only has a thin shell, may not have sufficient strength to withstand the rigours of insertion and the ceramic may fail from brittle fracture.

One proposal for addressing this by strengthening the rim of the ceramic acetabular cup is described in EP2174621 in which the prosthesis comprises an acetabular cup which is preferably formed from a ceramic and a metal band is applied around the outer circumference of the acetabular cup prosthesis and adjacent to the rim. The presence of the metal band provides additional strength to the ceramic prosthesis and holds it in hoop compression enabling it to be inserted in the acetabulum.

Whatever the composition and configuration of the acetabular cup prosthesis, an insertion tool is generally required to facilitate the insertion and correct positioning of the prosthesis in the pelvis. Cups which rely solely on a jamb fit with the bone require a greater force to be applied via the insertion tool than is the case with cemented polyethylene cups. This force is primarily to direct impact into the acetabulum but force may also be applied to adjust the angular position of the cup or to remove it if it has been position incorrectly.

In order to ensure that the required forces are accurately and safely applied to the cup, it is generally necessary that the insertion tool positively grips the cup. However, it is also important that the means by which the tool grips the cup does not impinge upon the outside of the cup in order that the tool does not become trapped between the shell and the pelvic bone. Further, as the wall thickness of the shell is generally kept to a minimum to minimise bone loss, the tool cannot generally grip the wall. Insertion tools are therefore usually designed so that their interaction with the acetabular cup does not negatively impact on the subsequent function of the acetabular hip prosthesis and its interaction with the pelvis. Further, it is general good practise to avoid gripping the internal surface of the cup as doing so could damage the bearing surface and also affect the wear performance of the prosthesis.

One means for connecting the acetabular cup prosthesis to an insertion tool is described in GB2323036. In the described arrangement, a cable is attached to the prosthesis and this is then used to connect the prosthesis to the tool. Once the prosthesis is located in the desired position, it may be necessary to cut the cable so that the tool and the prosthesis can be separated.

In an alternative arrangement, such as that described in EP1634552, an impaction cap is connected to the prosthesis by cables and the insertion tool then connects to the impaction cap. An alternative impaction cap system is described in EP1721586.

Other suggestions for enabling insertion tools to be connected to acetabular cup prostheses are described in, for example, US4677972, US590488, US5904688, US2004/0186586, and WO2014/108540. DE202008015356U1 discloses a hip prosthesis having an outer shell, insert and a plastic ring. US2013/282133A1 discloses an apparatus having an acetabular cup and a connection member. US2012/053699A1 discloses an acetabular cup component having a first shell, an annular ring and a second shell.

It is desirable to provide an alternative acetabular hip prosthesis which addresses some or all of the problems associated with the removal of the labrum during machining of the pelvic bone and thereby provides a joint which more closely mirrors the natural joint. In addition, it is desirable to provide an arrangement which allows for the practical use of acetabular cup prosthesis which have thin walls and/or which are made of materials such as ceramic which can be damaged during the forces required for impaction. It is also desirable to provide a prosthesis which can readily co-operate with an insertion tool. It is further desirable to provide an acetabular cup prosthesis which provides two or all of these requirements.

It is now proposed that some or all of these problems may be addressed by the use of an acetabular cup prosthesis including an engagement formation in the wall of the cup at or proximal to the rim thereof and which enables a lip member to be connected to the cup which in use may serve as an artificial labrum. The engagement formation may also be utilised to enable interaction with an introducer tool or an impaction cap.

Thus according to the present invention there is provided an acetabular cup prosthesis comprising: a cup having an inner cup surface and an outer cup surface, said inner cup surface meeting said outer cup surface at a rim of said cup prosthesis, wherein said inner cup surface, said outer cup surface or both said inner cup surface and said outer cup surface comprises a lip-member engagement formation located at or proximal to said rim, said lip-member engagement formation extending around at least a portion of the circumference of the cup prosthesis; and a lip member comprising a cup-surface engagement formation configured to cooperate with the lip-member engagement formation of the inner cup surface or the outer cup surface; characterised in that the lip member, once in position, is arranged to sit on and partially overlie the rim of the cup prosthesis to increase the height of the cup prosthesis, a free-end of the lip member extending upwardly and inwardly to form an artificial labrum.

It will be understood that in use, the inner cup surface will provide the articulation surface for the femoral head and the outer cup surface will be implanted into the pelvis.

In one arrangement, the engagement formation extends around the entire circumference. Where the engagement formation does not extend around the entire circumference, there may be two or more separate engagement formations. Where are a plurality of separate engagement formations are present, they may be equally spaced.

In one arrangement, the, or each, engagement formation is formed by a machined groove in the, or each, surface.

Where engagement formations are located on both the inner cup surface and the outer cup surface they may both extend around the entire circumference or one may extend around the entire circumference, while the other is around a portion thereof or there is a plurality of engagement formations.

In one arrangement, there may be more than one engagement formation on said inner cup surface, said outer cup surface or both said inner cup surface. For example there may be two formations on one surface each extending around substantially the entire ring and being located one below the other such that they are vertically spaced.

The location of the, or each, engagement formation may be located at any suitable position. In one arrangement, it may be located at from about 2 mm to about 6 mm from the rim of the cup.

The cup of the acetabular cup prosthesis may be formed from any suitable material. in one arrangement it may be formed from ceramic. Examples of suitable ceramic materials include alumina, zirconia, zirconia toughened alumina and silicon nitrides. For the purposes of this application, the term 'ceramic' should be construed as meaning not only true ceramic materials but also other materials which display ceramic-like properties. Ceramic-like properties for the purposes of the present invention are those where strength, stiffness, and rigidity, are similar to those of ceramics. Examples of suitable ceramic-like materials include glasses. In one arrangement, a metal coating may be provided on the ceramic.

In another arrangement, the acetabular cup prosthesis may be formed from metal. The metal cup may have a wear-resistant coating. The wear-resistant coating may have ceramic-like properties.

In one arrangement, the rim of the cup may be flat. The flat surface may be horizontal or it may be sloped such that the edge of the inner cup surface is higher or lower than the outer cup surface. However, in an alternative arrangement, it may be curved from the outside surface to the inside surface. The curvature may be concave, convex or substantially S-shaped.

The cup itself may be of any suitable configuration. Generally it will be substantially hemispherical. However, in one arrangement. the top edge may have a wave configuration.

In this arrangement, the cup may be handed such that a different wave configuration is required for each side of the body.

The lip member may be formed from any suitable material. In one arrangement, it may be formed from material which is softer than the material from which the cup is formed. The lip member may be formed from a polymeric material. Any suitable polymeric material may be used. Suitable materials include but are not limited to polyaryletherketones including polyetheretherketones, polyurethanes and other polymers having a suitable elastic modulus. Other materials such as nylon may be used. In one arrangement, the polymeric material may be reinforced such as with carbon fibres or particles.

The lip member, when connected to the cup via the respective engagement formations serves as an artificial labrum and therefore addresses the problems associated with prior art cups and the damage to the natural labrum by the machining of the pelvis to receive the acetabular cup prosthesis.

The lip member will generally be configured to extend around the circumference of the cup at or near the rim. It will be understood that where the engagement formation of the cup do not extend around the full circumference of the cup, the labrum may still extend around the complete circumference but will only be in engagement with the cup at the locations of the engagement formation.

The engagement formation may be of any suitable configuration. In general there may be a male configuration located on one of the cup prosthesis and the lip member and a cooperating female configuration located on the other of the cup prosthesis and the lip member.

In one arrangement, the engagement formation in one component may comprise a groove and that on the other component may comprise a flange which when the components are assembled the flange will be located in the groove such the lip member is held in position.

In another arrangement, the engagement formations on both components may be co-operating faces which have a transverse S-cross section.

The engagement formation may be formed by any suitable means. For example, it may be machined in the wall of the preformed cup. In an alternative arrangement, it may be incorporated in the wall(s) during manufacture thereof.

The lip member may be held in position by the interaction of the respective engagement means alone or in one arrangement, the lip member may be bonded in position using any suitable adhesive. In one alternative arrangement, the lip member may be formed in position on the cup prosthesis such as by injection moulding.

In one arrangement the lip member may be configured such that when it is in position on the outer cup surface, the profile of the outer cup surface and the outer surface of the lip member are contiguous. It will be understood that the lip member may additionally or alternatively be configured such that when it is in position on the inner cup surface, the profile of the inner cup surface and the inner surface of the lip member are contiguous. However, in one alternative arrangement, the lip member is of a size such that is slightly larger than the cup. This assists with initial fixation of the cup in the acetabulum.

The lip member may be of any suitable size and configuration. It may have the same configuration throughout, or it may be arranged such that the size is not constant throughout. Thus it may be configured such that it is wider and thinner in the position which will be in the anterior region when the cup is in position.

In its simplest form, the lip member once in position will simply sit on the rim of the cup to increase the height of the cup and provide it with an effective edge which has a lower modulus than the material from which the cup itself is formed. The presence of the lip not only provides the effect of an artificial labrum but may also provide compressive hoop stress to the cup which is advantageous where the cup is to be formed from ceramic.

The lip member extends upwardly and inwardly. Thus in one arrangement, said lip member may extend outwardly from the outer surface of the cup and be directed inwardly but not be connected to the inner surface of the cup such that a free edge of the lip member will be provided. In another arrangement, the lip member may extend upwardly continuing the line of the outer surface and then turn inwardly to provide the free edge. In a still further arrangement, the lip member may extend upwardly and inwardly from the inner cup surface.

In another arrangement, the free edge of the lip member may have shaping. In one arrangement, there may be a crenelated configuration. In one alternative arrangement, the free edge may have an undulated configuration. The size and spacing of the undulations may be of any suitable size and spacing.

Whichever configuration of lip member is adopted, the outer circumference of the lip member may include formations such as ribs. The presence of the formations will assist with initial fixation of the prosthesis into the pelvis and assist in preventing the rotation of the acetabular cup prosthesis when it is first implanted. Where ribs are used, any number may be present.

In one arrangement, a plurality of ribs may be spaced around the entire outer circumference. In one alternative arrangement, the ribs may be arranged in groups such that, for example, several groups of two, three, four or more ribs are spaced around the circumference.

In the arrangements in which the lip forms the artificial labrum discussed above, the material from which the lip member is formed is generally softer than the ceramic material from which the cup is formed. That is to say it has a lower elastic modulus. However, in other arrangements, it may be formed from material which is the same as or harder than the material from which the cup is to be formed. In one arrangement, it may be formed from metal.

The free edge of the lip member may be utilised to allow connection between the acetabular cup prosthesis and an insertion tool. In an alternative arrangement, the free edge may allow connection between the acetabular cup prosthesis and an impaction cap which in turn is connectable to an insertion tool. In this case, whilst a lip member having a lower elastic modulus that the material from which the cup is made may have sufficient strength either provided by the material per se or its configuration to enable effective interaction with an insertion tool or an impaction cap. However, it may be desirable to have the lip member formed from a material having a higher elastic modulus than the material from which the cup is formed.

Whatever material is used for the formation of the lip member, once the acetabular hip prosthesis has been located in the pelvis, the lip member may be removed by disengagement of the respective engagement formations. In one arrangement, it may be desirable to use a lip member having a higher elastic modulus for interconnecting it directly with the impaction tool and then remove it once the cup has been implanted. The higher elastic modulus lip member can then be removed and replaced with a lip member having a lower elastic modulus to provide the artificial labrum.

In one arrangement where the lip member is to be removed, it may not be continuous. Thus it may be discontinuous. It may be held in position by its own configuration. However, it may be held closed by means of an additional component such as a pin, clip, staple or the like. In another arrangement, the lip member may not be continuous but it may have an area of weakness which will break as the lip member is removed.

Where an impaction cap is to be used, it may be of any suitable configuration which allows it to engage with the free edge.

Whilst the lip member may be removed after insertion, it may be retained to act, in use, as an artificial labrum. Where the lip member is removed, it may be replaced by a further lip member which, in use, will serve as an artificial labrum.

Once the acetabular cup prosthesis has been implanted in the body, the tool and/or the impaction cap will be removed. A plug component may then be placed in connection with the free edge of the lip member to fill the space to the inner surface of the cup prosthesis. The plug component will generally be configured to be of a complementary shape to the lip member and its free edge. Thus, in the arrangement in which the free edge is undulating, the plug component will have complementary undulations.

Thus in another embodiment, the acetabular cup prosthesis comprises a plug component The plug component may be made of any suitable material. The material from which the plug component is manufactured may be the same or different to that from which the lip member is formed. In one arrangement it will be formed from a material having a lower elastic modulus to that from which the lip member is made such as polyurethane. However, polyaryletherketones or other medical grade polymers may also be used.

Whilst the lip member may be used to connect the cup to the introducer tool or the impaction cap, in another arrangement, the engagement formations on the or each cup surface may themselves be used for direct connection of the cup prosthesis to the introducer tool or to an impaction cap. In this arrangement, the tool or the impaction cap will be configured to have corresponding engagement formations to enable the cup prosthesis and the tool or cap to be interconnected.

Thus in a second aspect of the present invention there is provided a kit comprising the acetabular cup prosthesis of the above first aspect and an impaction cap or an introducer tool, said impaction cap or introducer tool comprising an engagement formation configured to cooperate with the engagement formation of the or each cup surface or the lip member such that when the engagement formation on the respective parts are in engagement, the impaction cap or introducer tool is attached to the cup prosthesis either directly or via the lip member.

Thus an introducer tool may, in use, be connected directly to the acetabular cup prosthesis or an impaction cap may, in use, be connected to the acetabular cup prostheses. In this latter arrangement an introducer tool will then generally be connection to an introducer tool. In one arrangement the impaction cap or introducer tool may sit on the upper surface of the cup prosthesis. In another arrangement, it may be configured to extend into a bowl of the cup prosthesis formed by the inner surface. In one arrangement in which the cap does extend into the bowl, the impaction cap or introducer tool may include a protuberance which in use interlocks with a recess or aperture in the bowl of the cup.

The impaction cap or introducer tool can be held in position during impaction by the interaction of the engagement formations. However, as the forces which are applied are substantial, it may be desirable to include additional fixation means to ensure that the impaction cap or introducer tool stays in position. Thus in one arrangement a wire arrangement such as circlip may be used although alternative means may be used. For example, the impaction cap or introducer tool may only engage with some of the engagement formations of the cup and the remainder could be used to enable a fixing means to be applied over the impaction cap to hold it in position.

Once the cup has been inserted, the impaction cap or introducer tool is removed. The cup may be left or the lip member and/or the plug component as described above may be applied.

It will be understood that the cup itself may be of any suitable configuration and may include, coatings or configurations to assist with initial fixation, ribs to restrict rotation and means to enable connection of the cup to an impaction cap or insertion tool where the lip or the engagement means of the present invention is not to be used for this purpose.

A further advantage of the present invention is that the engagement formation in the inner wall of the cup can be used to enable a liner to be attached to the cup. The liner can be made of any suitable material but will generally be polymeric. In one arrangement where there is an engagement formation on the inner cup surface, the liner will be provided with a cooperating engagement formation on the outer surface such that in use it engages with the inner surface.

However, alternatively or additionally, the liner may extend over the rim of the cup and extend at least partially over the rim and downwardly over the outer surface such that an engagement surface appropriately placed on the liner may engage with an engagement surface on the outer cup wall. The ability to introduce a polymeric liner may be advantageous in the event that the acetabular cup prosthesis were to fail. This is because inserting a liner may be preferable to removal of the cup which would otherwise be required in a revision operation. It may also be desirable to use a liner where it is desirable to use a smaller size replacement head on the femur.

In a still further arrangement, the engagement formation on the inner cup surface, the outer cup surface or both the inner and outer cup surfaces may be of a depth that they provide an area of weakness such that once the cup has been impacted, and the impaction cup and/or tool removed, the portion of the cup above the engagement portion can be removed by being sheared, snipped, torn or otherwise removed. It will be understood that in this configuration, the engagement formation will be located at the final height of the cup wall, that is to say the ultimate rim and the portion above the engagement formation is sacrificial Thus prior to implantation, the cup is larger in height than the final cup. The sacrificial portion may be a complete wall or it may comprise a plurality of tabs.

In this arrangement where there is a sacrificial portion, one or more additional engagement formations may be located at a point below the engagement formation which forms the point at which the sacrificial portion is removed. These one or more engagement formations will enable a hip member to be applied to the cup if desired once the sacrificial part has been removed.

In this arrangement, the so-called engagement formation whilst it may be used to engage an impaction tool or a hip may not actually be used to engage and may simply form an area of weakness.

According to an aspect not in accordance with the present invention there is provided a method of incorporating an acetabular cup prosthesis in which a cup according to the first aspect of the present invention has a lip member connected thereto via the respective engagement formations and the acetabular cup prosthesis is impacted into the pelvis.

In one arrangement, an impaction cap or an impaction tool is interconnected with the lip member prior to impaction. Once the cup has been impacted, the impaction cap or tool will be removed.

In another arrangement, once the cup has been impacted, the impaction cap or tool is removed, the hip member used during impaction is removed, and a second lip member is applied to act as an artificial labrum.

In a still further arrangement. once the cup has been impacted, a sacrificial portion of the cup may be removed at an engagement formation. In this arrangement a lip member may be applied once the sacrificial portion has been removed.

According to a further aspect not in accordance with the present invention, there is provided a method of incorporating an acetabular cup prosthesis in which a cup according to the first aspect of the present invention has an impaction cap connected thereto via the respective engagement formations and the acetabular cup prosthesis is impacted into the pelvis.

Once the cup has been impacted, the impaction cap or tool will be removed. A lip member may be applied.

According to a further aspect not in accordance with the present invention there is provided, an acetabular cup prosthesis comprising: an inner cup surface and an outer cup surface, said inner cup surface meeting said outer cup surface at a rim of said cup, wherein said inner cup surface, said outer cup surface or both said inner cup surface and said outer cup surface comprises an engagement formation
located proximal to said rim, said engagement formation extending around at least a portion of the circumference of the cup; said engagement formation being configured to allow, in use, interaction with an impaction cap or introducer tool.

The cup prosthesis of this aspect of the arrangement may be of any suitable configuration but will generally be as described above in connection with the first or second aspects of the present invention. Once the cup of this aspect has been introduced and the cap and/or tool removed, a lip member comprising an engagement formation configured to cooperate with the engagement formation of the cup may be connected to the cup. The lip member may be of any suitable configuration and may be of any of the arrangements described above in connection with the first or second aspects of the present invention.

The present invention will now be described, by way of example, with reference to the following drawings in which:
- Figure 1: is a perspective view of an acetabular cup prosthesis according to one aspect of the present invention;
- Figure 2: is a perspective view of the prosthesis of Figure 1 taken through a cross-section thereof;
- Figure 3: is an enlarged illustration of portion B of Figure 2;
- Figure 4: is a perspective view of an acetabular cup prosthesis according to a second aspect of the present invention;
- Figure 5: is a perspective view of the prosthesis of Figure 4 taken through a cross-section thereof;
- Figure 6: is an enlarged illustration of portion B of Figure 5;
- Figure 7: is a perspective view of an acetabular cup prosthesis according to a third aspect of the present invention;
- Figure 8: is a perspective view of the prosthesis of Figure 7 taken through a cross-section thereof;
- Figure 9: is an enlarged illustration of portion B of Figure 8;
- Figure 10: is a perspective view of an alternative lip member;
- Figure 11: is a perspective view of a section of the alternative lip member of Figure 10;
- Figure 12: is a perspective view of an acetabular cup prosthesis comprising the lip member of Figure 10;
- Figure 13: is a perspective view from above of one example of an impaction cap;
- Figure 14: is a view from above with of the impaction cap in position;
- Figure 15: is an enlarged view of part D of Figure 14;
- Figure 16: is a cross section on line A-A of Figure 14;
- Figure 17: is an enlarged view of part C of Figure 16;
- Figure 18: is a cross section on line B-B of Figure 16;
- Figure 19: is an enlarged view of part E of Figure 18;
- Figure 20: is an enlarged view of part F of Figure 18;
- Figure 21: is a perspective view from above with the plug component in position;
- Figure 22: is a schematic perspective view on cross section of Figure 21;
- Figure 23: is a view on line A-A of Figure 21
- Figure 24: is an enlarged view of section C of Figure 23;
- Figure 25: is a cross section on line B-B of Figure 23;
- Figure 26: is an enlarged view of part D of Figure 25;
- Figure 27: is a view from above of a further configuration of lip member with an impaction cap in position;
- Figure 28: is a side view of the arrangement of Figure 27 with a portion cut away;
- Figure 29: is an enlarged view of portion A of Figure 28;
- Figure 30: is a perspective view in cross-section on line B-B of Figure 27;
- Figure 31: is an alternative embodiment of the acetabular cup prosthesis of the present invention;
- Figure 32: is a perspective view of the acetabular cup prosthesis of Figure 31 including an impaction cap;
- Figure 33: is a view of the arrangement of Figure 32 from above;
- Figure 34: is a section view on line A-A of Figure 33;
- Figure 35: is a view of the acetabular cup of Figure 32 from above;
- Figure 36: is a section view on line B-B of Figure 35;
- Figure 37: is a perspective view of a still further embodiment of an acetabular cup prosthesis of the present invention;
- Figure 38: is an impaction cap for use in the cup of Figure 37;
- Figure 39: is a locking component for use with the impaction cap of Figure 38;
- Figure 40: is a view from above illustrating the impaction cap and locking member in place on the prosthesis of Figure 37;
- Figure 41: is a section view on line A-A of Figure 40;
- Figure 42: is an enlarged view of portion C from Figure 41;
- Figure 43: is a section view on line B-B of Figure 41;
- Figure 44: is an enlarged view of portion D of Figure 43; and
- Figure 45: is an illustration of the embodiment having a sacrificial portion.

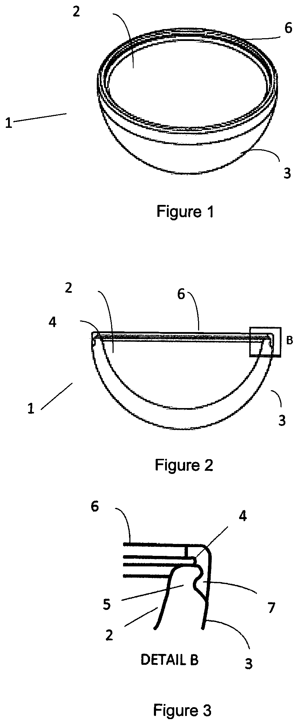

One arrangement of an acetabular cup prosthesis in accordance with the present invention is illustrated in Figures 1 to 3. The cup prosthesis 1 which may be formed from a ceramic comprises an inner cup wall 2 and an outer cup wall 3. The inner cup wall 2 and the outer cup wall 3 meet at rim 4. It will be understood that whilst the cup is described as having inner and outer walls meeting at a rim, the cup will be solid. In this arrangement, an engagement formation 5 is provided on the outer cup wall 3.

A lip member 6, having a co-operating engagement formation 7, is located such that engagement formation 7 interconnects with engagement formation 5 such that the lip member is held in place. In this arrangement, the lip member extends upwardly and then inwardly in a horizontal orientation. In use, this lip member which is formed of a low modulus material such as polyetheretherketone, serves as an artificial labrum.

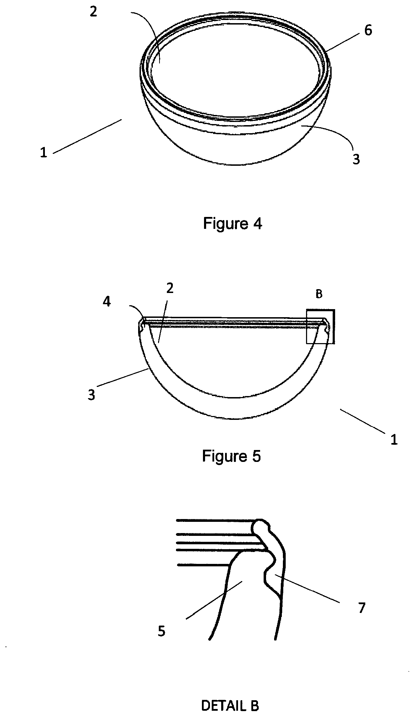

An alternative configuration of the lip member 6 is illustrated in Figures 4 to 6. In this arrangement, the lip member interconnects with the outer cup wall 3 via the respective engagement portions 7 and 5. In this arrangement, the lip member extends upwardly and then slopes inwardly. In use, this lip member which is formed of a low modulus material such as polyetheretherketone, serves as an artificial labrum.

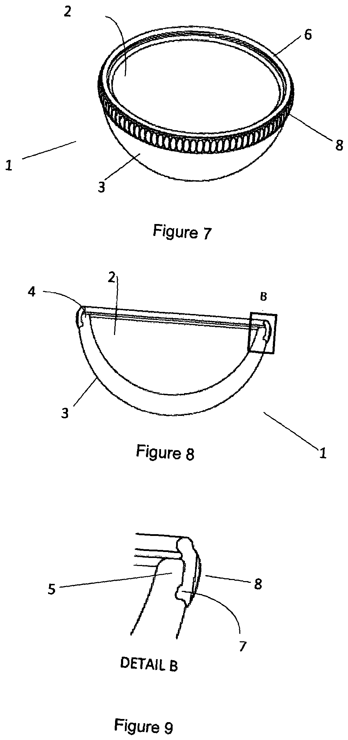

A still further alternative configuration of the lip member 6 is illustrated in Figures 7 to 9. In this arrangement, a plurality of ribs 8 is located on the outer surface of the lip member. These assist with the initial fixation of the acetabular cup prosthesis and serve to resist rotation of the cup once it is implanted. The ribs illustrated in this embodiment may be applied in other embodiments. It will be noted that the engagement formations 7 and 5 in this embodiment are of a different shape to those illustrated in Figures 2 and 3 or Figures 5 and 7. It will be understood that the engagement formations may be of any shape provided that they function and thus any illustrated configuration can be used with any embodiment.

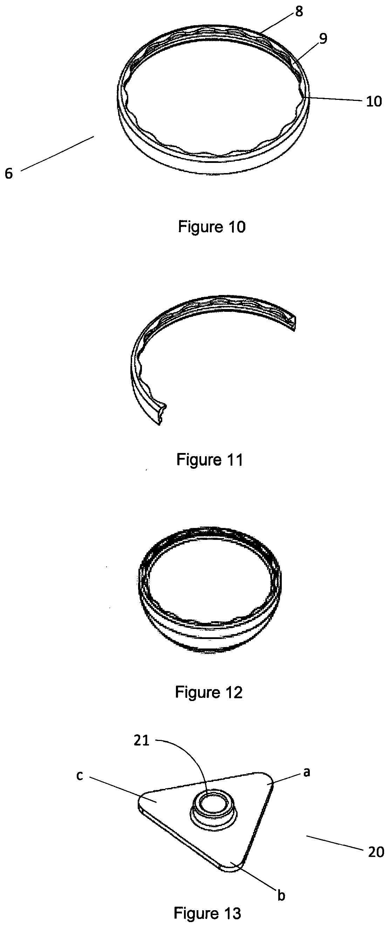

A still further arrangement of lip member 6 is illustrated in Figures 10 and 11. In this arrangement, the configuration of the lip member is similar to that of Figures 1 to 3 except that the inward portion rather than being of a consistent size has undulations such that it has a waved edge 8 having peaks 9 and troughs 10. Figure 11 illustrates a portion of the lip member 6 such that its profile can be seen in cross-section.

The undulations of the lip member as illustrated in Figures 10 and 11 enable an impaction cap to be readily interlocked with the acetabular cup. One example of a suitable impaction cap 20 is illustrated in Figure 13. Here the impaction cap is triangular in shape and the three lobes a, b, c, are sized such that they can pass between the troughs 10 of the wavy edge 6 of the lip 6 until the impaction cap is below the edge of the lip 6, It will then be rotated so that the lobes become situation beneath the peaks 9 of the waved edge such that the impaction cap 20 is held in position. In another alternative to increase strength a disc may be mounted beneath the triangular portion.

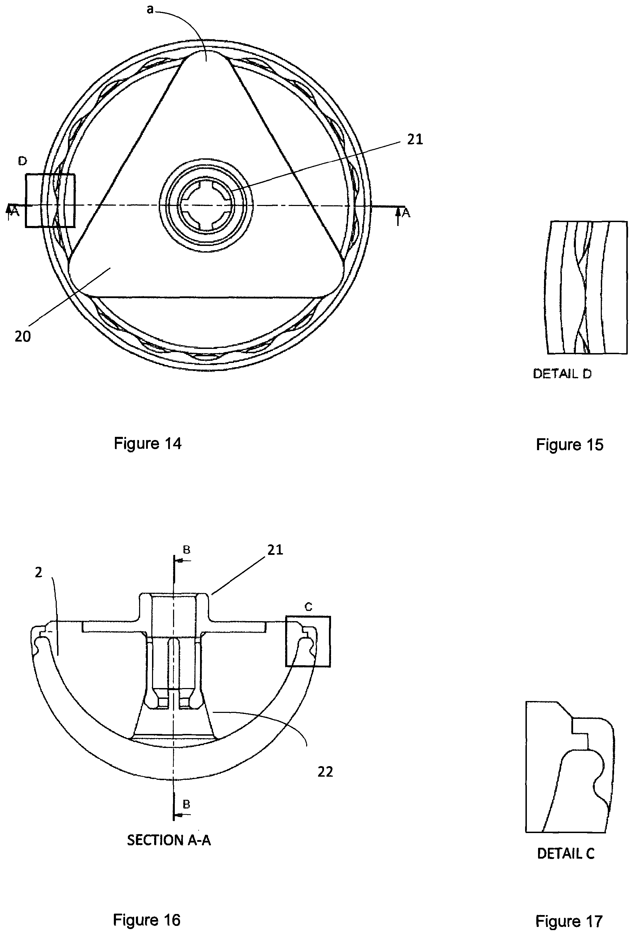

The cap 20 in position but prior to rotation is illustrated in Figure 14 and an enlarged portion is illustrated in Figure 15.

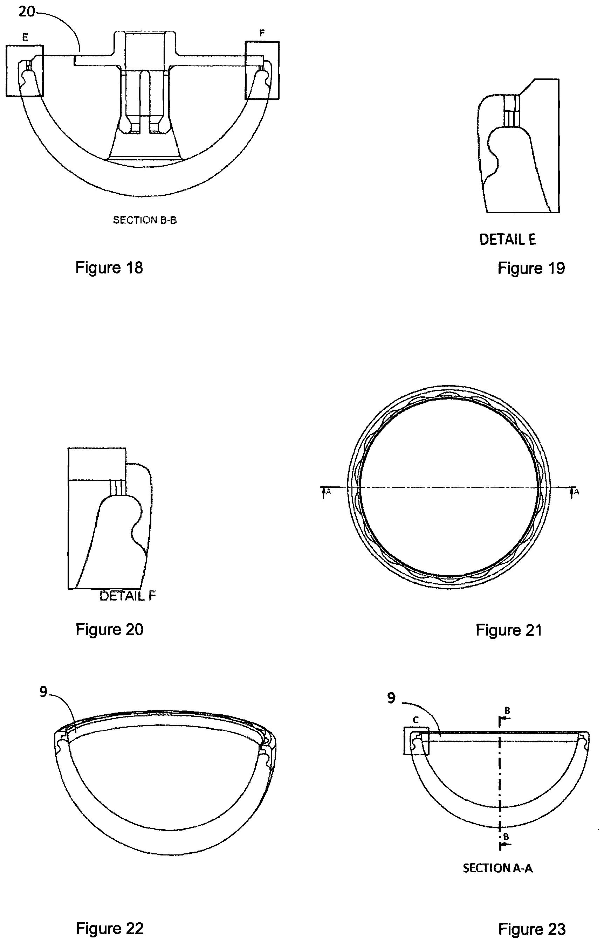

A central core 21 on the impaction cap enables it to be interlocked with an impaction tool (not shown). As illustrated in Figures 16 and 18, in one arrangement, the impaction cap 20 may include a central core 22 which in use extends into the cup and sits on the base of the inner cup surface 2 to provide additional support during impaction. Detail of the interaction between the impaction cap and the lip is illustrated in Figures 15, 17, 19 and 20.

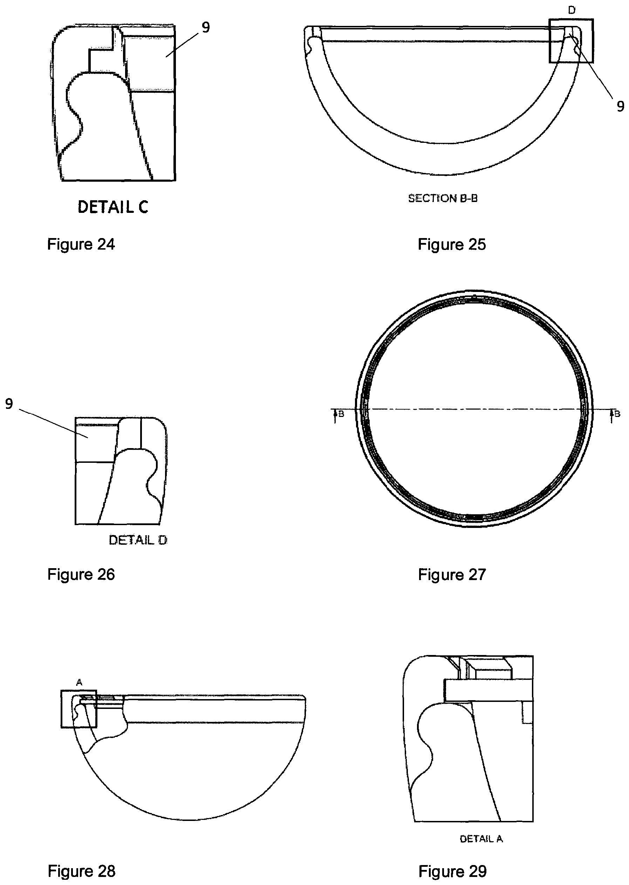

Once the acetabular cup prosthesis 1 has been introduced into the pelvis, the cap 20 is removed. A plug component 9 is then put in place. The plug component 9 also has an undulated configuration such that it when it is in position it fits against the undulations of the lip member 6. This is illustrated in detail in Figures 24 to 26.

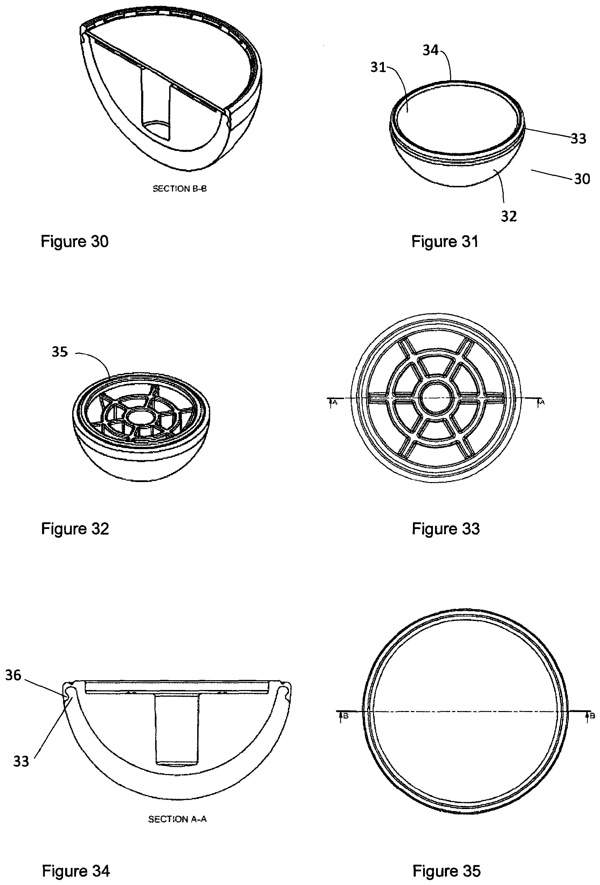

An alternative arrangement is illustrated in Figures 27 to 30. In this arrangement, rather than the lip member having undulations, it has a crenelated profile. The impaction cap may have cooperating crenels or it may have a plurality of tabs. The crenels on the cap or the tabs can be inserted through the spaces in the crenels in the lip member. When the cap is rotated it is locked under the crenels of the lip member. In an alternate arrangement, the cap may be a snap fit under the crenels of the lip member. As illustrated in Figure 30 the internal structure of the impaction cap may be different to that illustrated in Figure 18. However, it will be understood that the cap may be of any suitable configuration.

Figure 31 illustrates an alternative acetabular cup prosthesis 30 which has an inner cup surface 31 and an outer cup surface 32. Engagement formation 33 extends around the circumference of the outer cup surface 32 proximal to the rim 34 of the cup. In this embodiment, the impaction cap 35 does not engage with a lip member but rather extends over the rim 34 such that the corresponding impaction engagement formation 36 on the impaction cap engages with that on the outer cup wall such that the impaction cap is held in position.

In the illustrated arrangement the impaction cap has a webbed structure. However, it will be understood that this webbed structure for the impaction cap cab be utilised with the lip member arrangements provided that it is provided with cooperating lobes to interact with the lip member. Similarly a more solid impaction cap such as those discussed above can be used in this embodiment provided that it is configured to extend over the rim of the cup and engage with the engagement formations 33.

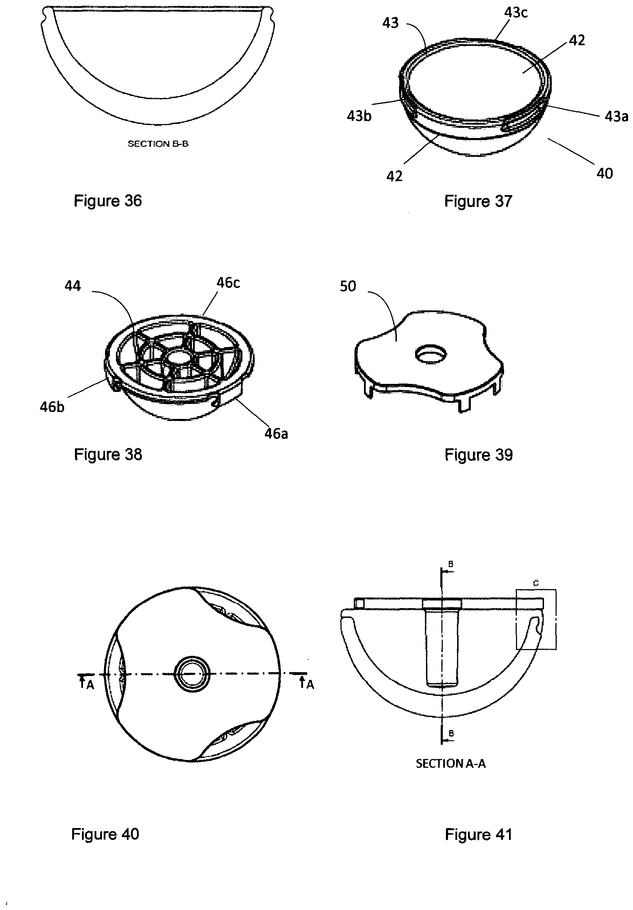

Once the acetabular cup prosthesis 30 is in position, the impaction cap will be removed to leave a cup as illustrated in Figures 35 and 36. However, it will be understood that other configurations of cup may also be used. A lip member may then be placed in position to provide an artificial labrum.

A still further arrangement is illustrated in Figure 37. In this arrangement, the acetabular cup which has an inner cup surface 41 and an outer cup surface 42 prosthesis 40 does not have engagement formations extending around the entire circumference. In contrast, there are three discrete areas in which the formations extend, 43a, 43b and 43c. In this arrangement, an impaction cap 44 is provided which extends over the rim 45 of the cup 40 and has three cooperating engagement formations 46a, 46b and 46c (not shown). As illustrated, the impaction cap 44 has a webbed configuration. However, it will be understood that any configuration of impaction cap.

The impaction cap 44 may be held in place simply by the interaction of the respective engagement formations, 43a and 46a, 43b and 46b and 43c and 46c. However, an additional locking member 50 may be used. Details of the components as assembled are illustrated in Figures 40 to 44.

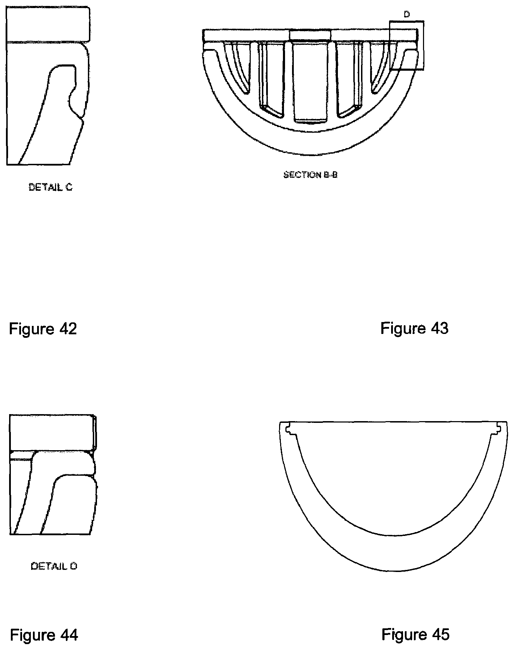

A further arrangement is illustrated in Figure 45. In this arrangement, the wall of the cup extends in a sacrificial portion 50 above the rim 51 of the cup. As illustrated, the engagement formation for engagement with the impaction cap or tool is on the inner wall surface 52 but it may alternatively or additionally be on the outer wall 53. The sacrificial portion may extend around the entire circumference or it may be made up of a plurality of tabs. Any number of tabs may be used but generally about 3 or 4 will suffice. Once the cup is impacted, the sacrificial portion may be removed.

## Claims

1. An acetabular cup prosthesis (1) comprising:
a cup having an inner cup surface (2) and an outer cup surface (3), said inner cup surface (2) meeting said outer cup surface (3) at a rim (4) of said cup prosthesis (1), wherein said inner cup surface (2), said outer cup surface (3) or both said inner cup surface (2) and said outer cup surface (3) comprises a lip-member engagement formation (5) located proximal to said rim (4), said lip-member engagement formation (5) extending around at least a portion of the circumference of the cup prosthesis (1); and
a lip member (6) comprising a cup-surface engagement formation (7) configured to cooperate with the lip-member engagement formation (5) of the inner cup surface (2) or the outer cup surface (3); **characterised in that** the lip member (6), once in position, is arranged to sit on and partially overlie the rim (4) of the cup prosthesis (1) to increase the height of the cup prosthesis (1), a free-end of the lip member (6) extending upwardly and inwardly to form an artificial labrum.

2. An acetabular cup prosthesis (1) according to Claim 1 wherein the cup is formed from ceramic.

3. An acetabular cup prosthesis (1) according to Claim 1 or 2 wherein the lip member (6) is formed from a polymeric material; and optionally
wherein the polymeric material is a polyaryletherketone or nylon.

4. An acetabular cup prosthesis (1) according to any one of Claims 1 to 3 wherein the lip-member engagement formation (5) on the cup prosthesis (1) is of a female configuration and the cup-surface engagement formation (7) on the lip member (6) is of a cooperating male configuration.

5. An acetabular cup prosthesis (1) according to any one of Claims 1 to 4 wherein the engagement formations on both components may be co-operating faces which have a transverse S-cross section.

6. An acetabular cup prosthesis (1) according to any one of Claims 1 to 5 wherein the respective engagement formations on the or each cup surface (2, 3) and lip member (6) are bonded together.

7. An acetabular cup prosthesis (1) according to any one of Claims 1 to 6 wherein the lip member (6) is configured such that when it is in position on the outer cup surface (3), the profile of the outer cup surface (3) and the outer surface of the lip member (6) are contiguous.

8. An acetabular cup prosthesis (1) according to any one of Claims 1 to 7 wherein the lip member (6) is configured such that when it is in position on the inner cup surface (2), the profile of the inner cup surface (2) and the inner surface of the lip member (6) are contiguous.

9. An acetabular cup prosthesis (1) according to any one of Claims 1 to 8 wherein the lip member (6) extends upwardly from the outer cup surface (3) and is directed inwardly to provide a free edge; and optionally
wherein the free edge of the lip member (6) comprises shaping; and optionally
wherein the shaping is crenalation or undulation.

10. An acetabular cup prosthesis (1) according to any one of Claims 1 to 9 wherein the outer circumference of the lip member (6) includes formations to restrict rotation of the acetabular cup prosthesis (1) when implanted; and optionally
wherein the formations are a plurality of ribs (8).

11. An acetabular cup prosthesis (1) according to any one of Claims 1 to 10 wherein the cup prosthesis (1) additionally comprises a plug component (9) which interlocks with the lip member (6); and optionally
wherein the plug component (9) is formed from a polymeric material; and
optionally
wherein the plug component (9) is formed from polyurethane.

12. An acetabular cup prosthesis (1) according to any one of Claims 1 to 11 wherein the cup prosthesis (1) includes a sacrificial portion above an engagement formation.

13. A kit comprising the acetabular cup prosthesis (1) of any one of Claims 1 to 12 and an impaction cap (20) or introducer tool, said impaction cap (20) or introducer tool comprising an engagement formation configured to cooperate with the engagement formation of the or each cup surface (2, 3) or the lip member (6).

14. A kit according to Claim 13 wherein the impaction cap (20) or introducer tool extends into a bowl of the cup prosthesis; and optionally
wherein the impaction cap (20) or introducer tool includes a protuberance which in use interlocks with a recess or aperture in the bowl of the cup prosthesis.

15. A kit according to any one of Claims 13 or 14, further comprising additional fixation to hold the impaction cap (20) in the acetabular cup prosthesis (1).

## Patentansprüche

1. Hüftgelenkpfannenprothese (1), umfassend:
eine Pfanne mit einer inneren Pfannenoberfläche (2) und einer äußeren Pfannenoberfläche (3), wobei die innere Pfannenoberfläche (2) die äußere Pfannenoberfläche (3) an einem Rand (4) der Pfannenprothese (1) trifft, wobei die innere Pfannenoberfläche (2), die äußere Pfannenoberfläche (3) oder sowohl die innere Pfannenoberfläche (2) als auch die äußere Pfannenoberfläche (3) eine Lippenelement-Eingriffsformation (5) umfassen, die proximal zu dem Rand (4) angeordnet ist, wobei sich die Lippenelement-Eingriffsformation (5) um mindestens einen Teil des Umfangs der Pfannenprothese (1) erstreckt; und
ein Lippenelement (6), umfassend eine Pfannenoberfläche-Eingriffsformation (7), die konfiguriert ist, um mit der Lippenelement-Eingriffsformation (5) der inneren Pfannenoberfläche (2) oder der äußeren Pfannenoberfläche (3) zusammenzuarbeiten; **dadurch gekennzeichnet, dass** das Lippenelement (6), sobald es in Position ist, so angeordnet ist, dass es auf dem Rand (4) der Pfannenprothese (1) sitzt und teilweise darüber liegt, um die Höhe der Pfannenprothese (1) zu erhöhen, wobei sich ein freies Ende des Lippenelements (6) nach oben und innen erstreckt, um ein künstliches Labrum zu bilden.

2. Hüftgelenkpfannenprothese (1) nach Anspruch 1, wobei die Pfanne aus Keramik gebildet ist.

3. Hüftgelenkpfannenprothese (1) nach Anspruch 1 oder 2, wobei das Lippenelement (6) aus einem Polymermaterial gebildet ist; und optional
wobei das Polymermaterial ein Polyaryletherketon oder Nylon ist.

4. Hüftgelenkpfannenprothese (1) nach einem der Ansprüche 1 bis 3, wobei die Lippenelement-Eingriffsformation (5) an der Pfannenprothese (1) eine weibliche Konfiguration aufweist, und die Pfannenoberflächen-Eingriffsformation (7) an dem Lippenelement (6) eine kooperierende männliche Konfiguration aufweist.

5. Hüftgelenkpfannenprothese (1) nach einem der Ansprüche 1 bis 4, wobei die Eingriffsformationen an beiden Komponenten zusammenwirkende Flächen sein können, die einen transversalen S-Querschnitt aufweisen.

6. Hüftgelenkpfannenprothese (1) nach einem der Ansprüche 1 bis 5, wobei die jeweiligen Eingriffsformationen auf der oder jeder Pfannenoberfläche (2, 3) und dem Lippenelement (6) miteinander verbunden sind.

7. Hüftgelenkpfannenprothese (1) nach einem der Ansprüche 1 bis 6, wobei das Lippenelement (6) so konfiguriert ist, dass, wenn es auf der äußeren Pfannenoberfläche (3) in Position ist, das Profil der äußeren Pfannenoberfläche (3) und die äußere Oberfläche des Lippenelements (6) zusammenhängend sind.

8. Hüftgelenkpfannenprothese (1) nach einem der Ansprüche 1 bis 7, wobei das Lippenelement (6) so konfiguriert ist, dass, wenn es auf der inneren Pfannenoberfläche (2) in Position ist, das Profil der inneren Pfannenoberfläche (2) und die innere Oberfläche des Lippenelements (6) zusammenhängend sind.

9. Hüftgelenkpfannenprothese (1) nach einem der Ansprüche 1 bis 8, wobei sich das Lippenelement (6) von der äußeren Pfannenoberfläche (3) nach oben erstreckt und nach innen gerichtet ist, um eine freie Kante bereitzustellen; und optional
wobei die freie Kante des Lippenelements (6) Formgebung umfasst; und optional wobei die Formgebung eine Krenalisierung oder Welligkeit ist.

10. Hüftgelenkpfannenprothese (1) nach einem der Ansprüche 1 bis 9, wobei der Außenumfang des Lippenelements (6) Formationen umfasst, um die Drehung der Hüftgelenkpfannenprothese (1) einzuschränken, wenn sie implantiert wird; und
optional, wobei die Formationen eine Mehrzahl von Rippen (8) ist.

11. Hüftgelenkpfannenprothese (1) nach einem der Ansprüche 1 bis 10,
wobei die Pfannenprothese (1) zusätzlich eine Stopfenkomponente (9) umfasst, die mit dem Lippenelement (6) verriegelt; und optional
wobei die Stopfenkomponente (9) aus einem Polymermaterial gebildet ist;
und optional
wobei die Stopfenkomponente (9) aus Polyurethan gebildet ist.

12. Hüftgelenkpfannenprothese (1) nach einem der Ansprüche 1 bis 11, wobei die Pfannenprothese (1) einen Opferabschnitt oberhalb einer Eingriffsformation enthält.

13. Kit, umfassend die Hüftgelenkpfannenprothese (1) nach einem der Ansprüche 1 bis 12 und eine Schlagkappe (20) oder ein Einführwerkzeug, wobei die Schlagkappe (20) oder das Einführwerkzeug eine Eingriffsformation umfasst, die oder das konfiguriert ist, um mit der Eingriffsformation der oder jeder Pfannenoberfläche (2, 3) oder des Lippenelements (6) zusammenzuarbeiten.

14. Kit nach Anspruch 13, wobei sich die Schlagkappe (20) oder das Einführwerkzeug in eine Schüssel der Pfannenprothese erstreckt; und optional
wobei die Schlagkappe (20) oder das Einführwerkzeug eine Ausstülpung aufweist, die bei Verwendung mit einer Aussparung oder Öffnung in der Schüssel der Pfannenprothese verriegelt.

15. Kit nach einem der Ansprüche 13 oder 14, ferner umfassend eine zusätzliche Fixierung zum Halten der Schlagkappe (20) in der Hüftgelenkpfannenprothese (1).

## Revendications

1. Prothèse de cupule acétabulaire (1) comprenant :
une cupule ayant une surface de cupule interne (2) et une surface de cupule externe (3), ladite surface de cupule interne (2) rencontrant ladite surface de cupule externe (3) au niveau d'un rebord (4) de ladite prothèse de cupule (1), dans laquelle ladite surface de cupule interne (2), ladite surface externe de cupule (3) ou à la fois ladite surface de cupule interne (2) et ladite surface de cupule externe (3) comprennent un profil d'emboîtement d'élément de lèvre (5) situé à proximité dudit rebord (4), ledit profil d'emboîtement d'élément de lèvre (5) s'étendant autour d'au moins une partie de la circonférence de la prothèse de cupule (1) ; et
un élément de lèvre (6) comprenant un profil d'emboîtement de surface de cupule (7) configuré pour coopérer avec le profil d'emboîtement d'élément de lèvre (5) de la surface de cupule interne (2) ou de la surface de cupule externe (3); **caractérisée en ce que** l'élément de lèvre (6), une fois en position, est agencé pour reposer sur et recouvrir partiellement le rebord (4) de la prothèse de cupule (1) afin d'augmenter la hauteur de la prothèse de cupule (1), une extrémité libre de l'élément de lèvre (6) s'étendant vers le haut et vers l'intérieur pour former un labrum artificiel.

2. Prothèse de cupule acétabulaire (1) selon la revendication 1, dans laquelle la cupule est formée à partir de céramique.

3. Prothèse de cupule acétabulaire (1) selon la revendication 1 ou 2, dans laquelle l'élément de lèvre (6) est formé à partir d'un matériau polymère ; et éventuellement
dans laquelle le matériau polymère est une polyaryléthercétone ou un nylon.

4. Prothèse de cupule acétabulaire (1) selon l'une quelconque des revendications 1 à 3, dans laquelle le profil d'emboîtement d'élément de lèvre (5) sur la prothèse de cupule (1) a une configuration femelle et le profil d'emboîtement de surface de cupule (7) sur l'élément de lèvre (6) a une configuration mâle coopérante.

5. Prothèse de cupule acétabulaire (1) selon l'une quelconque des revendications 1 à 4, dans laquelle les profils d'emboîtement sur les deux composants peuvent être des faces coopérantes qui ont une section transversale en S.

6. Prothèse de cupule acétabulaire (1) selon l'une quelconque des revendications 1 à 5, dans laquelle les profils d'emboîtement respectifs sur la ou sur chaque surface de cupule (2, 3) et l'élément de lèvre (6) sont liés ensemble.

7. Prothèse de cupule acétabulaire (1) selon l'une quelconque des revendications 1 à 6, dans laquelle l'élément de lèvre (6) est configuré de sorte que lorsqu'il est en position sur la surface de cupule externe (3), le profil de la surface de cupule externe (3) et de la surface externe de l'élément de lèvre (6) sont contigus.

8. Prothèse de cupule acétabulaire (1) selon l'une quelconque des revendications 1 à 7, dans laquelle l'élément de lèvre (6) est configuré de sorte que lorsqu'il est en position sur la surface de cupule interne (2), le profil de la surface de cupule interne (2) et de la surface interne de l'élément de lèvre (6) sont contigus.

9. Prothèse de cupule acétabulaire (1) selon l'une quelconque des revendications 1 à 8, dans laquelle l'élément de lèvre (6) s'étend vers le haut à partir de la surface de cupule externe (3) et est dirigé vers l'intérieur pour fournir un bord libre ; et éventuellement
dans laquelle le bord libre de l'élément de lèvre (6) comprend une mise en forme; et éventuellement
dans laquelle la mise en forme est une crénulation ou une ondulation.

10. Prothèse de cupule acétabulaire (1) selon l'une quelconque des revendications 1 à 9, dans laquelle la circonférence externe de l'élément de lèvre (6) comporte des formations pour restreindre la rotation de la prothèse de cupule acétabulaire (1) lorsqu'elle est implantée ; et éventuellement
dans laquelle les formations sont une pluralité de nervures (8).

11. Prothèse de cupule acétabulaire (1) selon l'une quelconque des revendications 1 à 10, dans laquelle la prothèse de cupule (1) comprend en outre un composant de bouchon (9) qui se verrouille avec l'élément de lèvre (6) ; et éventuellement
dans laquelle le composant de bouchon (9) est formé à partir d'un matériau polymère ; et éventuellement
dans laquelle le composant de bouchon (9) est formé à partir de polyuréthane.

12. Prothèse de cupule acétabulaire (1) selon l'une quelconque des revendications 1 à 11, dans laquelle la prothèse de cupule (1) comporte une partie sacrificielle au-dessus d'un profil d'emboîtement.

13. Kit comprenant la prothèse de cupule acétabulaire (1) selon l'une quelconque des revendications 1 à 12 et un capuchon d'impact (20) ou un outil d'introduction, ledit capuchon d'impact (20) ou ledit outil d'introduction comprenant un profil d'emboîtement configuré pour coopérer avec le profil d'emboîtement de la ou de chaque surface de cupule (2, 3) ou de l'élément de lèvre (6).

14. Kit selon la revendication 13, dans lequel le capuchon d'impact (20) ou l'outil d'introduction s'étend dans une cuvette de la prothèse de cupule ; et éventuellement
dans lequel le capuchon d'impact (20) ou l'outil d'introduction comporte une protubérance qui, en cours d'utilisation, se verrouille avec un évidement ou une ouverture dans la cuvette de la prothèse de cupule.

15. Kit selon l'une quelconque des revendications 13 ou 14, comprenant en outre une fixation supplémentaire pour maintenir le capuchon d'impact (20) dans la prothèse de cupule acétabulaire (1).
